# EUROPEAN PATENT APPLICATION

(11) **EP 1 523 981 A1**
(43) Date of publication of application: **20.04.2005**
(21) Application number: 03256439.5
(22) Date of filing: 13.10.2003
(51) Int. Cl.: A61K 31/137, A61K 9/22

(54) **Extended release formulations of venlafaxine**

(71) Applicant: Wyeth, Madison, New Jersey 07940 (US)
(72) Inventor: Rowley, Michael, Titchfield Fareham PO15 5EH (GB); Potts, Angela, Titchfield Hampshire PO14 4NT (GB); Sims, Edward Dr., Southampton Hampshire SO32 1DN (GB)
(74) Representative: Wileman, David Francis Dr.

(57) **Abstract**

This invention relates to an extended release tableted dosage formulation of the antidepressant venlafaxine hydrochloride or an optical form thereof having improved bioavailability.

## Description

This invention relates to extended release tablet formulations of venlafaxine for pharmaceutical or veterinary use, more particularly to extended or slow release tablet formulations containing venlafaxine and enantiomeric (R or S) forms of venlafaxine, to processes for preparing such formulations and their use.

### Background of the Invention

Venlafaxine, 1-[2-(dimethylamino)-1-(4-methoxyphenyl)ethyl]-cyclo-hexanol, is an important drug in the neuropharmacological armentarium used for treatment of depression and general anxiety disorders. Venlafaxine and the acid addition salts thereof are disclosed in US patent 4,535,186. Venlafaxine as the hydrochloride salt is presently administered orally to adults in compressed tablet form taken two or three times a day within the range 75 to 350 mg/day. EP 0639374 discloses the use of venlafaxine in the treatment of obesity, panic disorder, post-traumatic stress disorder, late luteal phase dysphoric disorder attention deficit disorder, Gilles de la Tourette syndrome, bulimia nervosa, generalised anxiety disorder or Shy Drager syndrome. EP-A-654264 teaches the use of venlafaxine in treating incontinence. U.S. Patent No. 5,506,270 (Upton et al.) claims venlafaxine's use in methods of treating hypothalamic amenorrhea in non-depressed women. US Patent No. 5,530,013 (Husbands et al.) claims venlafaxine's use for enhancing cognition.

It will be understood that the enantiomers may be separated from each other by standard resolution techniques known in the art. An example of such resolution techniques is that described by Yardley et al. for resolution of 1-[2-(Dimethylamino)-1-(4-methoxyphenyl)ethyl]cyclohexanol in J. Med. Chem, 1990, Vol. 33, No. 10, at page 2904.

The absolute configuration of the (+) enantiomer of venlafaxine was established as S by a single crystal X-ray analysis of the hydrobromide salt and the anomalous dispersion technique (Yardley et al., J. Med. Chem., 1990, 33, 2899).

U.S. Patents Nos. 5,788,986 (Dodman) and 5,554,383 (Dodman) teach and claim the veterinary use of serotonin reuptake inhibitors including (±)-, R- or S-venlafaxine in modifying the behavior of dogs.

In therapeutic dosing with venlafaxine hydrochloride immediate release tablets, rapid dissolution results in a rapid increase in blood plasma levels of the active compound shortly after administration followed by a decrease in blood plasma levels over several hours as the active compound is eliminated or metabolized, until sub-therapeutic plasma levels are approached after about twelve hours following administration, thus requiring additional dosing with the drug. With the plural daily dosing regimen, the most common side effect is nausea, experienced by about forty five percent of patients under treatment with venlafaxine hydrochloride (see Entsuahi, R. and Chitra, R. Psychopharm. Bul., Vol. 33(4) 671 1997). Vomiting also occurs in about seventeen percent of the patients.

To ameliorate the problem encapsulated sustained release formulations of venlafaxine hydrochloride have been developed; - see EP 0797991A1 published 1 October 1997 which discloses encapsulated venlafaxine sustained release formulations wherein microcrystalline cellulose and hydroxypropylmethylcellulose were used in making practical venlafaxine-containing spheroids. EP 0797991 A1 states that it was completely unexpected that an extended release formulation containing venlafaxine hydrochloride could be obtained because the hydrochloride of venlafaxine proved to be extremely water soluble.

EP 0797991 A1 further states that numerous spheroid formulations were prepared using different grades of microcrystalline cellulose and hydroxypropyl methylcellulose, different ratios of venlafaxine hydrochloride and filler, different binders such as polyvinylpyrrolidone, methylcellulose, water, and polyethylene glycol of different molecular weight ranges in order to find a formulation which would provide a suitable granulation mix which could be extruded properly. In the extrusion process, heat buildup occurred which dried out the extrudate so much that it was difficult to convert the extruded cylinders into spheroids. EP 0797991 A1 then states further that addition of hydroxypropylmethylcellulose 2208 to the venlafaxine hydrochloride-microcrystalline cellulose mix made production of spheroids practical.

WO 99/22724 published 14 May 1999 discloses an encapsulated extended release venlafaxine formulation comprising spheroids substantially free of hydroxypropylmethylcellulose.

WO 94/27589 published 8 December 1994 discloses an osmotic dosage form that delivers a drug by osmotic action over an extended period of time. The drug composition comprised of venlafaxine and hydroxypropylalkylcellulose is delivered by displacement composition.

Immediate release venlafaxine hydrochloride tablets are marketed by Wyeth-Ayerst Laboratories under the Effexor® trademark. An extended release formulation of venlafaxine hydrochloride salt is available as Effexor XR for use in humans which is in the form of a capsule (37.5, 75mg and 150 mg) for once daily dosing, typically in the range from 75 mg/day to 225 mg/day. From pharmacokinetic studies the bioavailability of venlafaxine from such formulations is in the order of 40-45%, (see Patat et al., J. Clin. Pharmacol., 38 256 1998). Furthermore such an encapsulated formulation is more difficult and more time consuming to manufacture than extended release tablets which would require less sophisticated machinery. Tablets have the added advantage that they can be made divisible so that the dose can be titrated more accurately.

There is a need for a tablet formulation of venlafaxine hydrochloride which provides improved bioavailability preferably with an extended release profile.

There is also a need for an extended release tablet formulation of venlafaxine hydrochloride which is simpler to manufacture than the filled capsule formulation.

We have surprisingly found that it is possible to prepare a sustained release tablet formulation which has demonstrated very high bioavailability of venlafaxine in *in vivo* experiments in animals.

Furthermore we have been able to prepare such a tablet with satisfactory physical properties for bulk manufacture and commercial use.

Accordingly this invention provides an oral extended release pharmaceutical composition in compressed tablet form comprising venlafaxine or a salt thereof, especially a highly water soluble salt, such as the hydrochloride, and a carboxyvinyl polymer.

More particularly this invention provides a pharmaceutical composition in compressed tablet form for oral administration comprising venlafaxine or a highly water soluble venlafaxine salt and a carboxyvinyl polymer, the amounts of carboxyvinyl polymer being such as to extend the release of the highly water soluble drug over a period of about 8 hours or more from administration, e.g. when tested by dissolution procedures as described herein using simulated gastrointestinal fluids.

Unless the context states otherwise, as used herein, reference to venlafaxine is to be taken to include reference to optical forms thereof. Similarly reference to 'pharmaceutical' or 'pharmaceutically', e.g., as used in the term pharmaceutically acceptable compositions or salts, is to be taken to include the veterinary equivalent.

Based on animal data the tablet formulations of this invention can provide, in a single daily dose, extended release of venlafaxine so that it is possible to maintain a steady state therapeutic blood serum level up to a twenty four hour period. In particular through administration of the venlafaxine formulation of this invention, there is provided a method for obtaining a flattened drug plasma concentration to time profile, thereby affording a tighter plasma therapeutic range control than can be obtained with multiple daily dosing. Accordingly this invention provides a method for eliminating the sharp peaks and troughs (hills and valleys) in blood plasma drug levels induced by multiple daily dosing with conventional immediate release venlafaxine hydrochloride tablets. In essence, the plasma levels of venlafaxine hydrochloride rise, after administration of the extended release formulations of this invention, for about four hours and then begin to fall through a protracted, substantially linear decrease from the peak plasma level for the remainder of the twenty four hour period, maintaining at least a threshold therapeutic level of the drug during the entire twenty-four period. Hence, in accordance with the use aspect of this invention, there is provided a method for moderating the plural blood plasma peaks and valleys attending the pharmacokinetic utilization of multiple daily rapid release tablet dosing with venlafaxine hydrochloride which comprises administering to a patient in need of treatment with venlafaxine hydrochloride, a one-a-day, extended release tablet formulation of venlafaxine hydrochloride and carbopol.

Resulting from the increase in bioavailability of venlafaxine in the compositions of this invention it should be possible to lower the daily dosage amount of active which for capsule formulations was typically between 75 and 225 mg/day.

Carboxyvinyl polymers used in the present invention are very high molecular weight acrylic acid polymers which are chemically cross-linked with polyalkenyl alcohols or divinyl alcohol which results in a polymer which is insoluble but swellable in water. Carboxyvinyl polymers are commercially available from B. F. Goodrich Company, Cleveland, Ohio, USA under the brand name Carbopol. Particularly preferred for use in the present invention is Carbopol 971P. Typically the carboxyvinylpolmer component comprises a sufficient amount to prolong release of the venlafaxine active over a period not less than 8 hours after oral administration and preferably from about 8 to 24 hours after administration in simulated gastrointestinal fluids.

By adjusting the amount of carboxyvinyl polymer component in the tablets of the present invention complete release of the active venlafaxine can be prolonged for 8 or more hours after administration. Alternatively, (or in addition) a functional coating, e.g. an enteric coat or a delay coat, may be applied to the tablet to extend further the release profile of the active.

Typically the carboxyvinyl polymer component comprises from about 4% to 40%w/w of the tablet, e.g., about 8% to about 40%w/w, preferably about 8% to 30%w/w. Most preferably the carboxyvinyl polymer is from about 10% to 20%w/w of the tablet, e.g., about 13%w/w.

Other rate controlling excipients, e.g. hydrophilic matrices such as HPMC or hydrophobic matrices such as ethyl cellulose, waxes or fats, may be used in conjunction with the carboxy vinyl polymer in which case the amount of carboxy vinyl polymer may be reduced from the values above.

The active ingredient may comprise about 10-90% w/w of the tablet, e.g., about 20-50% w/w of the tablet. Preferably the active comprises about 30-40% w/w of the tablet. Examples of the active ingredient in the fomulations of this invention include venlafaxine, (R)-venlafaxine, (S)-venlafaxine, or a highly water soluble salt thereof such as the hydrochloride acid salt.

In addition the tablet formulations of the present invention can also comprise standard excipients including but not limited to one or more of talc, calcium carbonate, microcystalline cellulose, powdered cellulose, dextrates, kaolin, mannitol, silicic acid, sorbitol, starch, pre-gelatinized starch, dicalcium phosphate, microcrystalline cellulose, lactose and starch. The excipient content is typically from about 25 to about 75% w/w of the tablet, e.g., about 28-60%, preferably about 33-55%, most preferably about 40 -50%.

Dibasic calcium phosphate (hydrated or anhydrous) used in the present invention are commercially available.

The tablets may also comprise a lubricant. Suitable lubricants include, but are not limited to, calcium stearate, magnesium stearate, mineral oil, glycerin, sorbitol, mannitol, polyethylene glycol, other glycols, stearic acid, sodium lauryl sulfate, talc, hydrogenated vegetable oil, zinc stearate, ethyl oleate, ethyl laureate, agar, or mixtures thereof. Preferably, the lubricant magnesium stearate is used to aid tablet manufacture.

Silicon dioxide or other flow aid may also be present.

In a further aspect of this invention a functional coating, e.g. an enteric coat or a delay coat, may be applied to the tablet to delay and/or extend further the release profile of the venlafaxine active. Examples of suitable coating polymers to provide delayed release are mixtures of Eudragit RS30D and Eudragit RL30D. By varying the ratio of Eudragit RS30D and Eudragit RL30D (e.g. typically in the range from about 8:1 to about 5:1 respectively) and the quantity of the coat applied (e.g. up to 13%w/w) it is possible to increase the delay period.

The tablets of the present invention can be prepared by standard tableting procedures, such as for example, dry granulation, wet granulation with binder, or spray granulation with binder in accordance with methods known in the art. For instance, the active ingredient may be sieved through a suitable sieve and blended with excipients until a uniform blend if formed. The dry blend may be screened and blended with magnesium stearate. The resulting powder blend may then be compressed into tablets of desired shape and size. Preferred granulation and coating techniques are described below:

### (a) Dry blend

All materials, except magnesium stearate, are screened, then mixed for 15-20 minutes in an appropriate sized blender. Magnesium stearate is screened then added to the blender and mixed for a further 2 minutes. This is then compressed to form tablets.

### (b) Wet granulation

The carbopol and venlafaxine hydrochloride are mixed for 20 minutes in an appropriate sized blender. A granule forms by the addition of an appropriate aqueous or organic solvent. When dry the granule is milled to an appropriate size. The granule is combined with calcium phosphate and mixed for 15-20 minutes in an appropriate sized blender. Screened magnesium stearate is added to the blend which is then mixed for a further 2 minutes. This is then compressed to form tablets.

### (c) Dry granulation

All materials, except magnesium stearate, are screened, then mixed for 15-20 minutes in an appropriate sized blender. Half the magnesium stearate is screened then added to the blender and mixed for a further 2 minutes. This is then compressed to form compacts which are subsequently are milled to an appropriate size. The granule is blended with the remaining screened magnesium stearate for 2 minutes. This is then compressed to form tablets.

The following Examples illustrate this invention:

### EXAMPLE 1

An extended release tablet of venlafaxine hydrochloride containing 75mg (base) was made according to the dry granulation manufacturing process described above with the following constituents:

| **Ingredient** | **Tablet Weight mg** | **Tablet Weight (%)** |
|---|---|---|
| Venlafaxine hydrochloride | 84.84* | 36.10 |
| Carbopol 971P** | 30.00 | 12.77 |
| Dibasic calcium phosphate | 115.93 | 49.33 |
| Colloidal silicon dioxide | 0.94 | 0.40 |
| Magnesium stearate | 3.29 | 1.40 |
| TOTAL | 235.00 | 100 |

| | | |
|---|---|---|
| * Equivalent to 75mg base | | |
| ** Carboxyvinylpolymer available from BF Goodrich, Pharmaceuticals Division | | |

### DISSOLUTION TESTING

Dissolution testing was carried out using USP Type 2 apparatus in either 900 ml water at 100 rpm or 900 ml simulated media at 50 rpm over a 24 hour period. With the simulated fluid, dissolution was carried out in 900 ml simulated gastric fluid USP (no enzymes) for the first 2 hours then the media was changed to 900 ml simulated intestinal fluid USP (no enzymes). Dissolution testing was carried out at 37 °C. A 5 ml sample was removed from the dissolution vessel at the required time interval and the venlafaxine hydrochloride content and therefore dissolution rate was determined by ulraviolet spectroscopy. The dissolution data presented is the mean of at least 3 tablets.

### RESULTS

Results obtained in the dissolution tests are shown in the following TABLE 1

**TABLE 1**

| % Venlafaxine hydrochloride released into simulated gastrointestinal fluid as a function of time. | |
|---|---|
| **Time** **(hours)** | **% venlafaxine hydrochloride released** |
| 0 | 0 |
| 0.5 | 24.3 |
| 1 | 34.5 |
| 1.5 | 42 |
| 2 | 48.2 |
| 3 | 66.2 |
| 4 | 71.4 |
| 8 | 84.3 |
| 12 | 92.6 |
| 18 | 95.5 |
| 24 | 97 |

The results in Table 1 show an extended release profile over >8 hours from administration.

### Bioavailability Analysis on Venlafaxine Sustained Release Tablets

### In vivo test results in dogs:

Each of 2 groups of 4 male beagle dogs received a 75mg dose of venlafaxine from two formulations in fed state.
- Dogs 1-4 received commercial sustained release capsule formulation (Effexor XR)
- Dogs 5-8 received sustained release venlafaxine HCl tablet formulation of Example 1

Mean Venlafaxine Bioavailability Results in Dogs (n=4)

| Pharmacokinetic parameter | FORMULATION | |
|---|---|---|
| | Effexor XR | Example 1 |
| AUC₀₋₂₄ (ng.h/ml) | 2130 | 8271 |
| Cₘₐₓ (ng/ml) | 137 | 788 |
| tₘₐₓ (h) | 5.5 | 3.9 |
| Relative Bioavailability^{a} | 31 % | 122% |

| | | |
|---|---|---|
| ^{a} Relative bioavailability to a solution dose taken from a previous dog study where the relative bioavailability of Effexor XR was shown to be 30%. | | |

Mean Free ODV Bioavailability Results in Dogs (n=4)

| Pharmokinetic parameter | Formulation | |
|---|---|---|
| | Effexor XR | Example 1 |
| AUC₀₋₂₄(ng.h/ml) | 216.8 | 406.8 |
| Cₘₐₓ (ng/ml) | 24.7 | 47.9 |
| tₘₐₓ (h) | 2.5 | 1.5 |

These results showed a marked increase in bioavailability in dogs for the carbopol formulation of this invention over the commercial EFFEXOR XR formulation.

### Human studies with formulation of Example 1

A 5-period crossover study was carried out with administration of Example 1 venlafaxine HCl/carbopol tablets and the marketed 75-mg venlafaxine XR capsule. Twenty (20) subjects enrolled in the study, and 19 subjects completed. One subject withdrew from the study after receiving the formulation of EXAMPLE 1, but did not receive the marketed XR capsule. The following two tables summarize the pharmacokinetic profile of venlafaxine and its active metabolite, O-desmethylvenlafaxine for each formulation:

**TABLE 2**

| Mean Venlafaxine Bioavailability Results in Humans | | |
|---|---|---|
| Pharmacokinetic parameter | FORMULATION | |
| | Effexor XR | Example 1 |
| AUC₀₋₂₄ (ng.h/ml) | 729 | 790 |
| Cₘₐₓ (ng/mL) | 38 | 65 |
| tₘₐₓ (h) | 5.9 | 4.4 |
| t_{1/2} (h) | 10.8 | 6.1 |

**TABLE 3**

| Mean Free ODV Bioavailability Results in Humans | | |
|---|---|---|
| Pharmocokinetic parameter | Formulation | |
| | Effexor XR | Example 1 |
| AUC₀₋₂₄ (ng.h/ml) | 2578 | 2631 |
| Cₘₐₓ (ng/mL) | 83 | 120 |
| tₘₐₓ (h) | 10.4 | 6.8 |
| t_{1/2} (h) | 15.0 | 11.2 |

These results showed an increase in bioavailability in humans for the carbopol formulation of this invention over the commercial EFFEXOR XR formulation.

### EXAMPLE 2

An extended release tablet of venlafaxine hydrochloride containing 75mg (base) was made according to the dry granulation manufacturing process described in Example 1 with the constituents shown below:

| **Ingredient** | **Tablet Weight mg** | **Tablet Weight (%)** |
|---|---|---|
| Venlafaxine hydrochloride | 84.81 * | 36.40 |
| Carbopol 971P | 22.37 | 9.60 |
| Dibasic calcium phosphate | 122.56 | 52.60 |
| Colloidal silicon | 0.93 | 0.40 |
| Magnesium stearate | 2.33 | 1.00 |
| TOTAL | 233.00 | 100 |

| | | |
|---|---|---|
| * Equivalent to 75mg base | | |

### Dissolution Testing/Results

Dissolution testing was carried out as described in Example 1 and gave the results shown in TABLE 4:

**TABLE 4**

| % Venlafaxine hydrochloride released into simulated gastrointestinal fluid as a function of time (t) | |
|---|---|
| **Time** | **% venlafaxine hydrochloride released** |
| 0 | 0 |
| 0.5 | 28.4 |
| 1 | 40.3 |
| 1.5 | 48.7 |
| 2 | 55.5 |
| 3 | 71.7 |
| 4 | 78.3 |
| 8 | 89.8 |
| 12 | 94.5 |
| 18 | 96.1 |
| 24 | 98 |

### EXAMPLE 3

An extended release tablet of venlafaxine hydrochloride containing 75mg (base) was made according to the dry granulation manufacturing process described in Example 1 with the following constituents:

| **Ingredient** | **Tablet Weight mg** | **Tablet Weight (%)** |
|---|---|---|
| Venlafaxine hydrochloride | 84.84* | 36.10 |
| Carbopol971P | 37.60 | 16.00 |
| Dibasic calcium phosphate | 108.33 | 46.10 |
| Colloidal silicon | 0.94 | 0.40 |
| Magnesium stearate | 3.29 | 1.40 |
| TOTAL | 235.00 | 100 |

| | | |
|---|---|---|
| * Equivalent to 75mg base | | |

### Dissolution Testing/Results

Dissolution testing was carried out as described in Example 1 and gave the results shown in Table 5:

**TABLE 5**

| % Venlafaxine hydrochloride released into simulated gastrointestinal fluid as a function of time. | |
|---|---|
| **Time** | **% venlafaxine hydrochloride released** |
| 0 | 0 |
| 0.5 | 22.8 |
| 1 | 32.5 |
| 1.5 | 39.4 |
| 2 | 45.2 |
| 3 | 65.2 |
| 4 | 67.7 |
| 8 | 78.1 |
| 12 | 85.8 |
| 18 | 94.1 |
| 24 | 96.8 |

### EXAMPLE 4

An extended release tablet of venlafaxine hydrochloride containing 225mg (base) was made according to the dry blend manufacturing process described above with the following constituents:

| **Ingredient** | **Tablet Weight mg** | **Tablet Weight (%)** |
|---|---|---|
| Venlafaxine hydrochloride | 254.8* | 36.40 |
| Carbopol 971P | 140.0 | 20.00 |
| Dibasic calcium phosphate | 298.2 | 42.60 |
| Magnesium stearate | 7.0 | 1.00 |
| TOTAL | 700.00 | 100 |

| | | |
|---|---|---|
| * Equivalent to 225mg base | | |

### Dissolution Testing/Results

Dissolution testing was carried out as described in Example 1 and gave the results shown below in TABLE 6:

**TABLE 6**

| % Venlafaxine hydrochloride released in water as a function of time. | |
|---|---|
| **Time** | **% venlafaxine hydrochloride released** |
| 0 | 0 |
| 0.5 | 15.9 |
| 1 | 22.0 |
| 1.5 | 26.4 |
| 2 | 29.8 |
| 4 | 40.2 |
| 8 | 55.0 |
| 12 | 66.2 |
| 18 | 78.9 |
| 24 | 89.5 |

### EXAMPLE 5

An extended release tablet of venlafaxine hydrochloride containing 225mg (base) was made according to the dry blend manufacturing process described in above with the following constituents:

| **Ingredient** | **Tablet Weight mg** | **Tablet Weight (%)** |
|---|---|---|
| Venlafaxine hydrochloride | 254.8* | 36.40 |
| Carbopol 971P | 112.7 | 16.10 |
| Dibasic calcium phosphate | 325.5 | 46.5 |
| Magnesium stearate | 7.0 | 1.00 |
| TOTAL | 700.00 | 100 |

| | | |
|---|---|---|
| * Equivalent to 225mg base | | |

### Dissolution Testing/Results

Dissolution testing was carried out as described in Example 1 and gave the results shown in TABLE 7:

**TABLE 7**

| % Venlafaxine hydrochloride released into water as a function of time. | |
|---|---|
| **Time** | **% venlafaxine hydrochloride released** |
| 0 | 0 |
| 0.5 | 18.5 |
| 1 | 26.4 |
| 1.5 | 32.1 |
| 2 | 36.6 |
| 4 | 49.3 |
| 8 | 66.9 |
| 12 | 78.9 |
| 18 | 90.1 |
| 24 | 98.7 |

### EXAMPLE 6

An extended release tablet of venlafaxine hydrochloride containing 225mg (base) was made according to the dry blend manufacturing process described above with the following constituents:

| **Ingredient** | **Tablet Weight mg** | **Tablet Weight (%)** |
|---|---|---|
| Venlafaxine hydrochloride | 254.8* | 39.20 |
| Carbopol 971P | 112.45 | 17.30 |
| Dibasic calcium phosphate | 211.25 | 32.5 |
| Magnesium stearate | 6.5 | 1.00 |
| HPMC 2208 100,000 SR | 65.0 | 10 |
| TOTAL | 650.00 | 100 |

| | | |
|---|---|---|
| * Equivalent to 225mg base | | |

### Dissolution Testing/Results

Dissolution testing was carried out as described in Example 1 and gave the results shown in TABLE 8:

**TABLE 8**

| % Venlafaxine hydrochloride released into water as a function of time | |
|---|---|
| **Time** | **% venlafaxine hydrochloride released** |
| 0 | 0 |
| 0.5 | 18.3 |
| 1 | 25.6 |
| 1.5 | 30.7 |
| 2 | 34.9 |
| 4 | 46.7 |
| 8 | 64.0 |
| 12 | 76.6 |
| 18 | 88.2 |
| 24 | 96.4 |

### EXAMPLE 7

An extended release tablet of venlafaxine hydrochloride containing 225mg (base) was made according to the wet granulation manufacturing process described above with the following constituents:

| **Ingredient** | **Tablet Weight mg** | **Tablet Weight (%)** |
|---|---|---|
| Venlafaxine hydrochloride | 254.8* | 36.40 |
| Carbopol971P | 140.0 | 20.00 |
| Dibasic calcium phosphate | 298.2 | 42.6 |
| Magnesium stearate | 7.0 | 1.0 |
| TOTAL | 700.00 | 100 |

| | | |
|---|---|---|
| * Equivalent to 225mg base | | |

### Dissolution Testing/Results

Dissolution testing was carried out as described in Example 1 and gave the results shown in Table 9.

**TABLE 9**

| % Venlafaxine hydrochloride released into water as a function of time | |
|---|---|
| **Time** | **% venlafaxine hydrochloride released** |
| 0 | 0 |
| 0.5 | 16 |
| 1 | 26 |
| 1.5 | 35 |
| 2 | 42 |
| 4 | 63 |
| 8 | 81 |
| 12 | 95 |
| 18 | 107 |

## Claims

1. An oral extended release pharmaceutical or veterinary composition in compressed tablet form comprising an active selected from venlafaxine, or an enantiomeric form of venlafaxine; or a water soluble salt thereof and a carboxyvinyl polymer.

2. A composition as claimed in claim 1 wherein the amount of carboxyvinyl polymer is from about 4% to 40%w/w of the tablet.

3. A composition as claimed in claim 1 wherein the amount of carboxyvinyl polymer is from about 8% to 30%w/w of the tablet.

4. A composition as claimed in claim 1 wherein the amount of carboxyvinyl polymer is from about 10% to 20%w/w of the tablet.

5. A composition as claimed in claim 1 wherein the amount of carboxyvinyl polymer is about 13%w/w of the tablet.

6. A composition as claimed in any one of Claims 1 to 5 in which the active comprises about 10-90% w/w of the tablet.

7. A composition as claimed in any one of Claims 1 to 5 in which the active comprises about 20-50% w/w of the tablet.

8. A composition as claimed in any one of Claims 1 to 5 in which the active comprises about 30-40% w/w of the tablet.

9. A composition as claimed in any one of Claims 1 to 8 in which the active is venlafaxine, (R)-venlafaxine, (S)-venlafaxine, or a highly water soluble salt thereof.

10. A composition as claimed in anyone of claims 1 to 8 in which the active is venlafaxine hydrochloride acid salt.

11. A composition as claimed in anyone of claims 1 to 10 wherein the tablet also comprises one or more excipients.

12. A composition as claimed in claim 11 wherein the amount of excipient comprises about 25-75%w/w of the tablet.

13. A composition as claimed in claim 11 wherein the amount of excipient comprises about 28-60%w/w of the tablet.

14. A composition as claimed in claim 11 wherein the amount of excipient comprises about 33-55%w/w of the tablet.

15. A composition as claimed in claim 11 wherein the amount of excipient comprises about 40-50%w/w of the tablet.

16. A composition as claimed in any one of Claims 11 to 15 in which the excipient is selected from one or more of dicalcium phosphate, microcrystalline cellulose, lactose and starch.

17. A composition as claimed in any one of Claims 11 to 15 in which the excipient is dicalcium phosphate.

18. A composition according to any one of the previous claims in which the tablet has a coating to delay or extend the release of said active.

19. A composition according to claim 18 in which the tablet has a functional coating comprising a mixture of Eudragit RS30D and Eudragit RL30D.

20. A composition according to claim 19 in which the Eudragit RS30D and Eudragit RL30D are in the ratio from about 8:1 to about 5:1.

21. A composition as claimed in any one of claims 1 to 20 in which the amount of carboxyvinyl polymer is such that the substantially complete release of active occurs in a period not less than about 8 hours after oral administration in simulated gastrointestinal fluids.

22. A composition as claimed in any one of claims 1 to 20 wherein the release of active occurs in a period from about 8 hours to 24 hours after administration.

23. A composition as claimed in anyone of claims 1 to 22 in which the carboxyvinyl polymer is Carbopol 971P.
